# EUROPEAN PATENT APPLICATION

(11) **EP 1 917 964 A1**
(43) Date of publication of application: **07.05.2008**
(21) Application number: 06782123.1
(22) Date of filing: 26.07.2006
(51) Int. Cl.: A61K 31/438, A61K 9/06, A61K 9/70, A61K 47/14, A61P 27/02, C07D 491/107

(54) **PERCUTANEOUSLY ABSORBABLE OPHTHALMIC PREPARATION**

(30) Priority: 26.07.2005 JP 2005216442
(71) Applicant: Senju Pharmaceutical Co., Ltd., Osaka-shi, Osaka 541-0046 (JP)
(72) Inventor: ISOWAKI, A., c/o SENJU PHARMACEUTICAL CO., LTD., Kobe-shi, Hyogo 651-2241 (JP); OHTORI, A., c/o SENJU PHARMACEUTICAL CO., LTD., Kobe-shi, Hyogo 651-2241 (JP)
(74) Representative: Weber, Thomas
(86) International application number: PCT/JP2006/315246
(87) International publication number: WO 2007/013661

(57) **Abstract**

The present invention provides a percutaneously absorbable ophthalmic preparation that permits the retention of a therapeutically effective concentration of a heterocyclic spiro compound and a salt thereof for promoting lacrimation, and that produces less adverse reactions such as miosis. Specifically, the present invention provides a percutaneously absorbable ophthalmic preparation comprising as an active ingredient a heterocyclic spiro compound represented by the general formula (I): 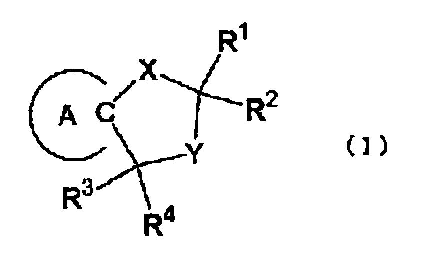 [wherein the symbols have the same definitions as those given in the description] or a pharmaceutically acceptable salt thereof.

## Description

### Technical Field

The present invention relates to a percutaneously absorbable ophthalmic preparation comprising a heterocyclic spiro compound or a salt thereof, to be administered to the eyelid skin surface to promote lacrimation.

### Background Art

The dry eye is a pathologic condition caused due to a decrease in the amount of tear secreted or a change in tear composition, and may produce corneal or conjunctival epithelial erosions, foreign body sensation and the like. As examples of dry eyes, dry eyes associated with ocular diseases such as Sjogren's syndrome, Stevens-Johnson syndrome, blepharitis, and meibomitis, dry eyes associated with VDT (visual display terminal) work, wearing of contact lenses and the like, and the like can be mentioned. A usual way of preventing or treating dry eyes is tear replenishment by administration of artificial tear, or administration of an ophthalmic solution containing a viscoelastic substance such as hyarulonic acid or chondroitin sulfate. However, these methods are only transiently effective, requiring frequent administration. Furthermore, generally, ophthalmic solutions are often supplemented with a preservative, and sometimes produce adverse reactions due to the preservative.

Dry eyes can be prevented and treated with a drug that promotes lacrimation. Among the drugs that promote lacrimation, for example, muscarinic receptor agonists such as heterocyclic spiro compounds and pilocarpine are known. Muscarinic receptors, also called muscarinic acetylcholine receptors, are involved in neurotransmission and stimulatory action in the organs under the control of the parasympathetic nerves, and are distinguished from nicotinic receptors in the autonomic ganglia and ganglionic junctions -(also called nicotinic acetylcholine receptors). Muscarinic receptors are also 7-pass transmembrane G protein coupled receptors, and are classified into five subtypes from M1 to M5. Muscarinic receptors are also widely distributed in ocular tissue, and are involved in a broad range of functions, including lacrimating action, lens curvature alteration, and aqueous humor outflow dynamics.

Amid this situation, it has been reported that a heterocyclic spiro compound which is a muscarinic receptor agonist is used as an eyedrop or an ocular ointment for the treatment of dry eyes (JP-A-2003-63964). However, instilling a muscarinic receptor agonist directly into an eye induces tear secretion, but also poses a problem of causing adverse reactions such as miosis. Therefore, there is a demand for a method of reducing adverse reactions of heterocyclic spiro compounds which are muscarinic receptor agonists, which is effective in the treatment of dry eyes.

Regarding preparations for the treatment of dry eyes, other than eyedrops, in reports of a therapeutic method for dry eyes by administering a nicotinic acetylcholine receptor agonist (see the pamphlet for International Publication No. 01/080844 and the description for US Patent 6,277,855) and a lacrimation-promoting composition comprising an ingredient that activates PAR-2 (see JP-A-2001-181208 and the description for US Patent Published Application 2003/203849), it is reported that these active ingredients can be used as percutaneously absorbable preparations.

Also reported as a percutaneously absorbable preparation for the treatment of ocular disease is, for example, a percutaneously absorbable ophthalmic adhesive preparation having a drug-containing layer consisting of a base matrix containing a drug to be delivered to the lens and the posterior segment of eye, including vitreous body, choroid and retina, and a percutaneous absorption enhancer (see the pamphlet for International Publication No. 01/26648 and the description for European Patent 1221315). Furthermore, a percutaneous absorption system for reducing intraocular pressure comprising the pilocarpine base, which is a muscarinic receptor agonist, or a salt thereof, has been reported (see JP-T-HEI-8-509716 and the description for US Patent 5,869,086). However, these reports lack a description of the promotion of lacrimation.

That is, in any of the aforementioned references, there is no description or disclosure of a lacrimation promoting preparation of the percutaneously absorbable type comprising a heterocyclic spiro compound which is a muscarinic receptor agonist.

Also reported is a percutaneously absorbable preparation for the treatment of ocular disease to be applied to a skin surface including the anterior surface of eyelid to deliver a therapeutic drug for ocular disease, without being mediated by systemic blood flow, to the topical tissue of the eye by skin permeation (pamphlet for International Publication No. 04/064817), but this report does not describe a percutaneously absorbable preparation that promotes lacrimation, nor is there any description of the suppression of adverse reactions such as miosis.

### Disclosure of the Invention

It is an object of the present invention to provide a percutaneously absorbable ophthalmic preparation that permits the retention of a therapeutically effective concentration of a heterocyclic spiro compound or a salt thereof for promoting lacrimation, and that produces less adverse reactions such as miosis.

It is another object of the present invention to provide a percutaneously absorbable ophthalmic preparation that exhibits a more sustained lacrimation promoting effect when administered to the eyelid skin surface to allow a heterocyclic spiro compound or a salt thereof to transfer substantially percutaneously directly from the eyelid skin to the topical tissue of the eye, rather than transferring to the topical tissue of the eye via systemic circulation, than when administered to a site other than the eyelid skin surface.

The present inventors diligently investigated with the aim of accomplishing the above-described objects, as a result found that by administering a percutaneously absorbable ophthalmic preparation comprising a heterocyclic spiro compound or a salt thereof to the eyelid skin surface, it is possible to sustainedly promote lacrimation, with less adverse reactions such as miosis, conducted further investigations based on this finding, and developed the present invention.

Accordingly, the present invention is as follows:
(1) A percutaneously absorbable ophthalmic preparation comprising as an active ingredient a heterocyclic spiro compound represented by the general formula (I): 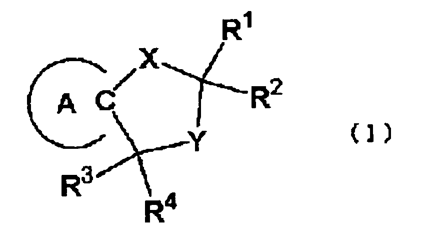 [wherein the ring A indicates a piperidine ring whose nitrogen atom is optionally substituted by a lower alkyl group, a lower alkanoyl group, or a lower alkoxycarbonyl group, provided that the nitrogen atom of the piperidine ring and an optionally chosen non-spiro-bound carbon atom optionally form a bridge via a lower alkylene group,
   X represents an oxygen atom or a sulfur atom,
   Y represents a carbonyl group, a thiocarbonyl group, a group represented by the formula: 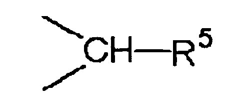 a group represented by the formula: 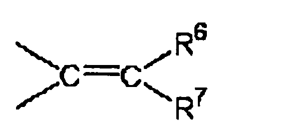 or a group represented by the formula: 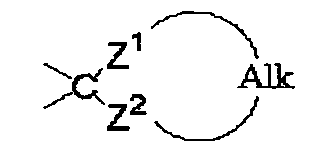 R¹, R² and R³ are the same or different, and each represents a hydrogen atom or a lower alkyl group,
   R⁴ represents a hydrogen atom, a lower alkyl group, a carboxy group, a lower alkoxycarbonyl group, or a lower alkanoyl group,
   R⁵ represents a halogen atom, a hydroxyl group, a mercapto group, a lower alkoxy group, a lower alkylthio group, a lower alkanoyloxy group, or a lower alkanoylthio group,
   R⁶ and R⁷ are the same or different, and each represents a hydrogen atom or a lower alkyl group,
   Z¹ and Z² are the same or different, and each represents an oxygen atom or a sulfur atom,
   Alk represents a lower alkylene group]
   or a pharmaceutically acceptable salt thereof.
(2) The percutaneously absorbable ophthalmic preparation described in (1), which is administered to the eyelid skin surface.
(3) The percutaneously absorbable ophthalmic preparation described in (1) or (2), which promotes lacrimation.
(4) The percutaneously absorbable ophthalmic preparation described in any one of (1) to (3), wherein the ring A in the general formula (I) is a piperidine ring whose nitrogen atom is substituted by a lower alkyl group, X is an oxygen atom, Y is a carbonyl group or a vinylidene group, R¹ is a lower alkyl group, and each of R², R³ and R⁴ is a hydrogen atom.
(5) The percutaneously absorbable ophthalmic preparation described in any one of (1) to (3), wherein the active ingredient is (-)-(S)-2,8-dimethyl-3-methylene-1-oxa-8-azaspiro[4,5]decane L-tartrate monohydrate.
(6) The percutaneously absorbable ophthalmic preparation described in any one of (1) to (5), wherein the amount of the active ingredient contained is 0.1 to 40% by weight.
(7) The percutaneously absorbable ophthalmic preparation described in any one of (1) to (6), further comprising an absorption enhancer.
(8) The percutaneously absorbable ophthalmic preparation described in (7), wherein the amount of the absorption enhancer contained is 1 to 60% by weight.
(9) The percutaneously absorbable ophthalmic preparation described in (7) or (8), wherein the absorption enhancer is isopropyl myristate.
(10) The percutaneously absorbable ophthalmic preparation described in any one of (1) to (9), which is an adhesive preparation.
(11) The percutaneously absorbable ophthalmic preparation described in any one of (1) to (9), which is an ointment.
(12) The percutaneously absorbable ophthalmic preparation described in any one of (1) to (9), which is a gel.
(13) A commercial package comprising the percutaneously absorbable ophthalmic preparation described in any one of (1) to (12) and a written matter stating an explanation about the use of the preparation for the promotion of lacrimation and that the preparation produces less miosis than preparations for instillation.
(14) A method of promoting lacrimation by administering to the eyelid skin surface a percutaneously absorbable ophthalmic preparation comprising as an active ingredient a heterocyclic spiro compound represented by the general formula (I): 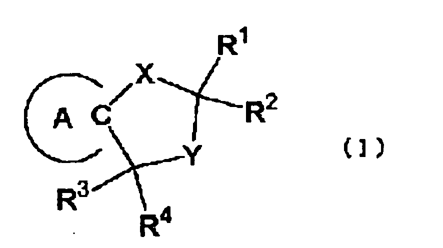 [wherein the ring A represents a piperidine ring whose nitrogen atom is optionally substituted by a lower alkyl group, a lower alkanoyl group, or a lower alkoxycarbonyl group, provided that the nitrogen atom of the piperidine ring and an optionally chosen non-spiro-bound carbon atom optionally form a bridge to via a lower alkylene group,
   X represents an oxygen atom or a sulfur atom,
   Y represents a carbonyl group, a thiocarbonyl group, a group represented by the formula: 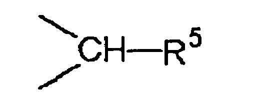 a group represented by the formula: 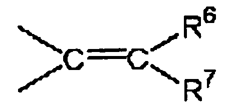 or a group represented by the formula: 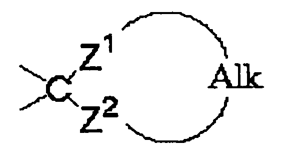 R¹, R² and R³ are the same or different, and each represents a hydrogen atom or a lower alkyl group,
   R⁴ represents a hydrogen atom, a lower alkyl group, a carboxy group, a lower alkoxycarbonyl group, or a lower alkanoyl group, R⁵ represents a halogen atom, a hydroxyl group, a mercapto group, a lower alkoxy group, a lower alkylthio group, a lower alkanoyloxy group, or a lower alkanoylthio group,
   R⁶ and R⁷ are the same or different, and each represents a hydrogen atom or a lower alkyl group,
   Z¹ and Z² are the same or different, and each represents an oxygen atom or a sulfur atom,
   Alk represents a lower alkylene group]
   or a pharmaceutically acceptable salt thereof.
(15) Use of a heterocyclic spiro compound represented by the general formula (I): 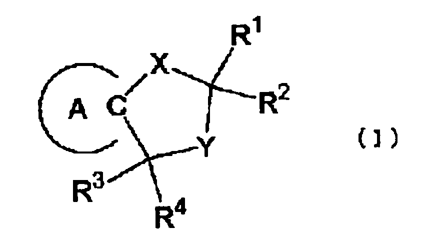 [wherein the ring A represents a piperidine ring whose nitrogen atom is optionally substituted by a lower alkyl group, a lower alkanoyl group, or a lower alkoxycarbonyl group, provided that the nitrogen atom of the piperidine ring and an optionally chosen non-spiro-bound carbon atom optionally form a bridge via a lower alkylene group,
   X represents an oxygen atom or a sulfur atom,
   Y represents a carbonyl group, a thiocarbonyl group, a group represented by the formula: 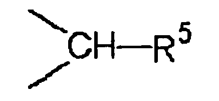 a group represented by the formula: 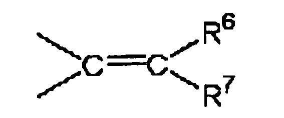 or a group represented by the formula: 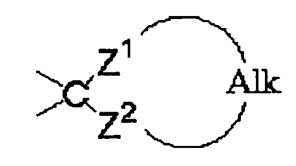 R¹, R² and R³ are the same or different, and each represents a hydrogen atom or a lower alkyl group,
   R⁴ represents a hydrogen atom, a lower alkyl group, a carboxy group, a lower alkoxycarbonyl group, or a lower alkanoyl group, R⁵ represents a halogen atom, a hydroxyl group, a mercapto group, a lower alkoxy group, a lower alkylthio group, a lower alkanoyloxy group, or a lower alkanoylthio group,
   R⁶ and R⁷ are the same or different, and each represents a hydrogen atom or a lower alkyl group,
   Z¹ and Z² are the same or different, and each represents an oxygen atom or a sulfur atom,
   Alk represents a lower alkylene group] or a pharmaceutically acceptable salt thereof, for producing a percutaneously absorbable ophthalmic preparation.

According to the present invention, a percutaneously absorbable ophthalmic preparation comprising a heterocyclic spiro compound or a salt thereof, capable of promoting lacrimation when administered to the eyelid skin surface, and a method of promoting lacrimation by administering the percutaneously absorbable preparation to the eyelid skin surface, can be provided. Furthermore, the percutaneously absorbable ophthalmic preparation of the present invention is a highly safe preparation that produces very few adverse reactions such as miosis, which are observed with the use of preparations for direct administration to eyes, such as ophthalmic solutions.

The percutaneously absorbable ophthalmic preparation of the present invention is also a preparation that exhibits a more sustained lacrimation promoting effect when administered to the eyelid skin surface to allow a heterocyclic spiro compound or a salt thereof to transfer substantially percutaneously directly from the eyelid skin to the topical tissue of the eye, rather than transferring to the topical tissue of the eye via systemic circulation, than when administered to a site other than the eyelid skin surface.

Therefore, because the percutaneously absorbable ophthalmic preparation of the present invention is capable of sustainedly promoting lacrimation, with less adverse reactions such as miosis, it is useful as a prophylactic/therapeutic agent for dry eyes, for example, dry eyes associated with ocular diseases such as lacrimal hyposecretion, dry eye syndrome, Sjogren's syndrome, keratoconjunctivitis sicca, Stevens-Johnson syndrome, blepharitis, and meibomitis, dry eyes associated with VDT (visual display terminal) work and wearing of contact lenses, and the like.

### Brief Description of the Drawings

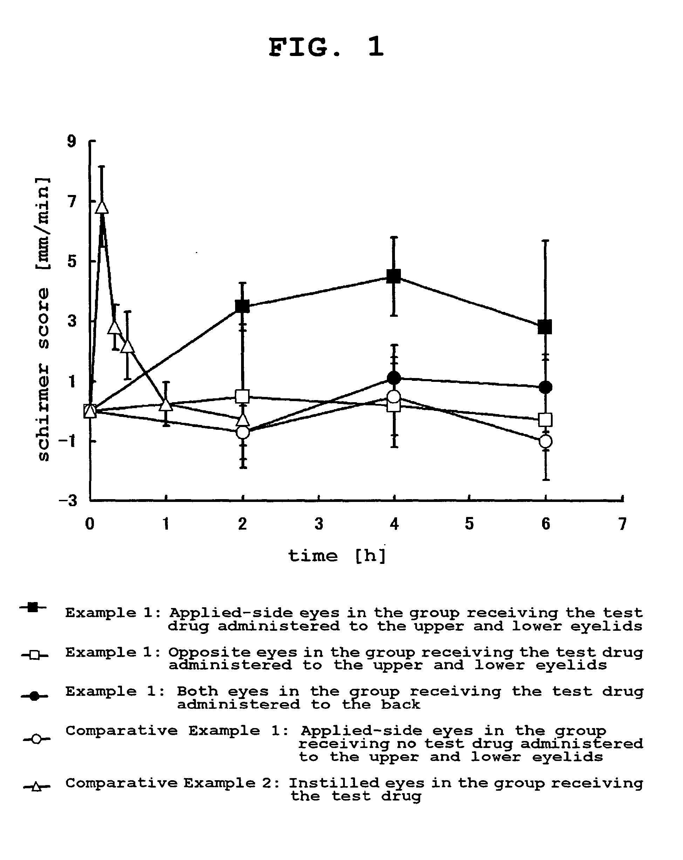

FIG. 1 is a graph showing the increases in the amount of tear secreted after administration of an adhesive preparation containing Compound A (Example 1) (applied eyes in the upper and lower eyelid administration group, n=3; opposite eyes in the upper and lower eyelid administration group, n=3; both eyes in the back administration group, n=6), an adhesive preparation not containing Compound A (Comparative Example 1) (applied eyes in the upper and lower eyelid administration group, n=3) and an ophthalmic solution containing Compound A (Comparative Example 2) (10 minutes to 2 hours after instillation, n=8) in Test Example 1. Each value indicates a mean±standard error.

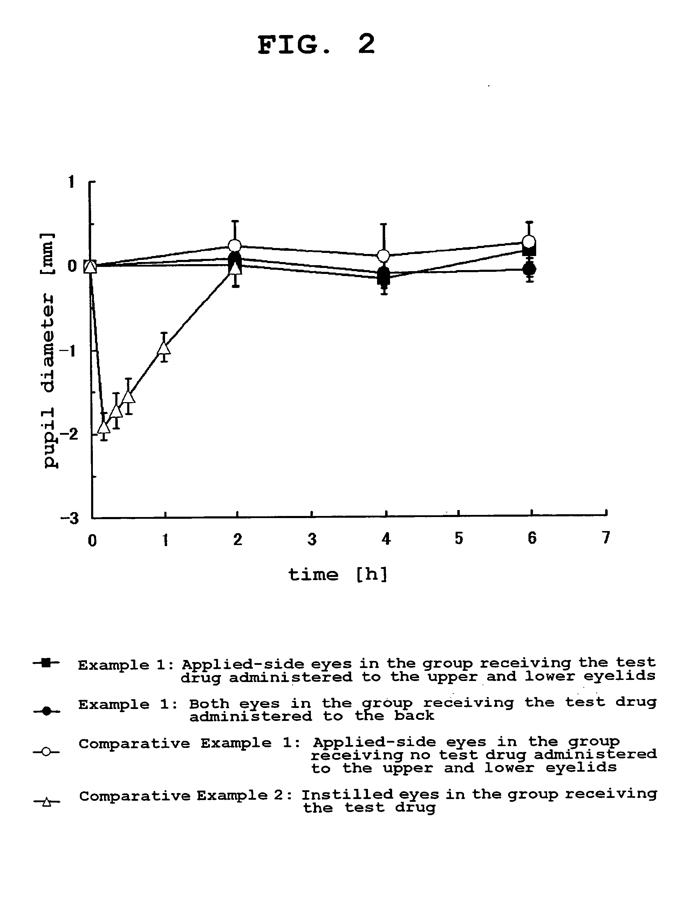

FIG. 2 is a graph showing the changes in pupil diameter after administration of an adhesive preparation containing Compound A (Example 1) (applied eyes in the upper and lower eyelid administration group, n=3; both eyes in the back administration group, n=6), an adhesive preparation not containing Compound A (Comparative Example 1) (applied eyes in the upper and lower eyelid administration group, n=3) and an ophthalmic solution containing Compound A (Comparative Example 2) (10 minutes to 2 hours after instillation, n=8) in Test Example 2. Each value indicates a mean±standard error.

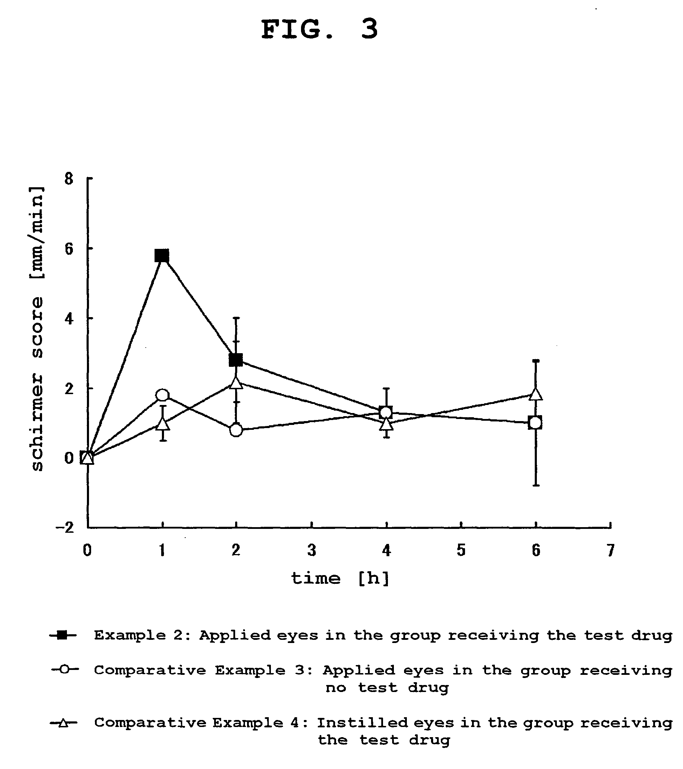

FIG. 3 is a graph showing the increases in the amount of tear secreted after administration of an ointment containing Compound A (Example 2) (1 hour after application, n=2; 2 to 6 hours after application, n=3), an ointment not containing Compound A (Comparative Example 3) (n=2), and an ophthalmic solution containing Compound A (Comparative Example 4) (n=3) in Test Example 3. Each value indicates a mean±standard error.

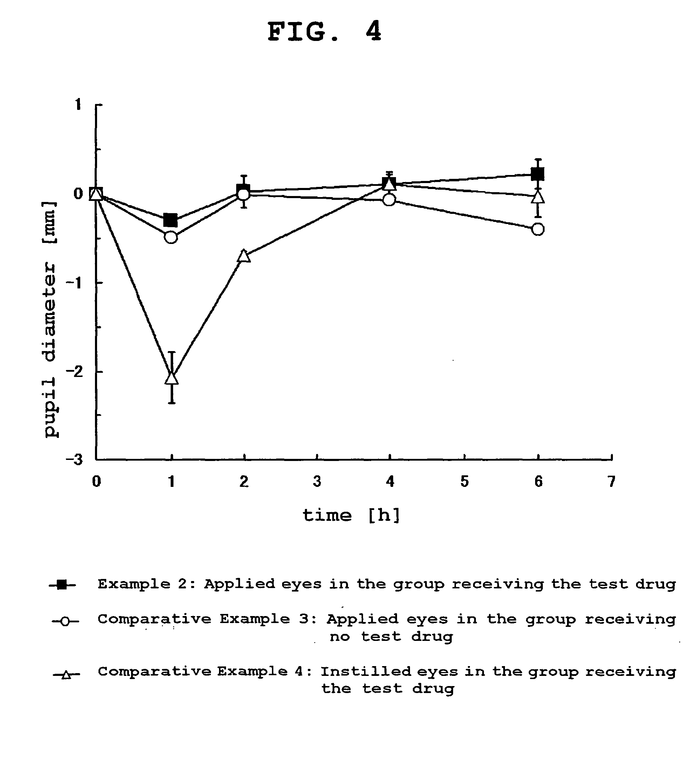

FIG. 4 is a graph showing the changes in pupil diameter after administration of an ointment containing Compound A (Example 2) (1 hour after application, n=2; 2 to 6 hours after application, n=3), an ointment not containing Compound A (Comparative Example 3) (n=2), and an ophthalmic solution containing Compound A (Comparative Example 4) (n=3) in Test Example 4. Each value indicates a mean±standard error.

### Best Mode for Carrying out the Invention

The percutaneously absorbable ophthalmic preparation of the present invention (hereinafter also simply referred to as the percutaneously absorbable preparation of the present invention) contains as an active ingredient a heterocyclic spiro compound represented by the general formula (I) above or a pharmaceutically acceptable salt thereof (hereinafter also referred to as the present compound).

In the definition for the general formula (I) above, the term "lower" means a linear or branched carbon chain having 1 to 6 carbon atoms unless otherwise specified. Therefore, as examples of "a lower alkyl group", methyl group, ethyl group, propyl group, isopropyl group, butyl group, isobutyl group, sec-butyl group, tert-butyl group, pentyl group, isopentyl group, neopentyl group, tert-pentyl group, 1-methylbutyl group, 2-methylbutyl group, 1,2-dimethylpropyl group, hexyl group, isohexyl group, 1-methylpentyl group, 2-methylpentyl group, 3-methylpentyl group, 1,1-dimethylbutyl group, 1,2-dimethylbutyl group, 2,2-dimethylbutyl group, 1,3-dimethylbutyl group, 2,3-dimethylbutyl group, 3,3-dimethylbutyl group, 1-ethylbutyl group, 2-ethylbutyl group, 1,1,2-trimethylpropyl group, 1,2,2-trimethylpropyl group, 1-ethyl-1-methylpropyl group, 1-ethyl-2-methylpropyl group and the like can be mentioned. As examples of "a lower alkanoyl group", formyl group, acetyl group, propionyl group, butyryl group, isobutyryl group, valeryl group, isovaleryl group, pivaloyl group, hexanoyl group and the like can be mentioned. As examples of "a lower alkoxycarbonyl group", methoxycarbonyl group, ethoxycarbonyl group, propoxycarbonyl group, isopropoxycarbonyl group, butoxycarbonyl group, isobutoxycarbonyl group, sec-butoxycarbonyl group, tert-butoxycarbonyl group, pentyloxycarbonyl group, isopentyloxycarbonyl group, neopentyloxycarbonyl group, tert-pentyloxycarbonyl group, hexyloxycarbonyl group and the like can be mentioned. As examples of "a lower alkoxy group", methoxy group, ethoxy group, propoxy group, isopropoxy group, butoxy group, isobutoxy group, sec-butoxy group, tert-butoxy group, pentyloxy (amyloxy) group, isopentyloxy group, tert-pentyloxy group, neopentyloxy group, 2-methylbutoxy group, 1,2-dimethylpropoxy group, 1-ethylpropoxy group, hexyloxy group and the like can be mentioned. As examples of "a lower alkylthio group", methylthio group, ethylthio group, propylthio group, isopropylthio group, butylthio group, sec-butylthio group, tert-butylthio group, pentylthio group, neopentylthio group, 2-methylbutylthio group, 1,2-dimethylpropylthio group, 1-ethylpropylthio group, hexylthio group and the like can be mentioned. As examples of "a lower alkanoyloxy group", formyloxy group, acetoxy group, propionyloxy group, butyryloxy group, isobutyryloxy group, valeryloxy group, isovaleryloxy group, pivaloyloxy group, hexanoyloxy group and the like can be mentioned. As examples of "a lower alkanoylthio group", formylthio group, acetylthio group, propionylthio group, butyrylthio group, isobutyrylthio group, valerylthio group, isovalerylthio group, pivaloylthio group, hexanoylthio group and the like can be mentioned.

As examples of the bridge optionally formed by the nitrogen atom of the piperidine ring represented by the ring A and an optionally chosen non-spiro-bound carbon atom via a lower alkylene group, 1-azabicyclo[2.2.1]heptane ring, 1-azabicyclo[2.2.2]octane ring (quinucridine ring), 1-azabicyclo[3.2.2]nonane ring, 1-azabicyclo[3.3.1]heptane ring, 1-azabicyclo[2.2.1]octane ring, 1-azabicyclo[3.3.1]nonane ring, 7,7-dimethyl-1-azabicyclo[2.2.1]heptane ring and the like can be mentioned. As examples of the halogen atom, fluorine atom, chlorine atom, bromine atom, iodine atom and the like can be mentioned. As examples of the "lower alkylene group" represented by Alk, ethylene group, trimethylene group, 1- or 2-methylethylene group, 1- or 2-ethylethylene group, 1- or 2-propylethylene group, 1- or 2-isopropylethylene group, 1- or 2-butylethylene group, 1,2-dimethylethylene group, 1,2-diethylethylene group, 1-ethyl-2-methylethylene group, 2-ethyl-1-methylethylene group, 1- or 2- or 3-methyltrimethylene group, 1- or 2- or 3-ethyltrimethylene group, 1- or 2- or 3-propyltrimethylene group, 1- or 2- or 3-isopropyltrimethylene group, 1,2- or 1,3- or 2,3-dimethyltrimethylene group, 1,2- or 1,3- or 2,3-diethyltrimethylene group, 1,2,3-trimethyltrimethylene group, 1,2,3-triethylmethylene group and the like can be mentioned.

As examples of the pharmaceutically acceptable salt of a compound represented by the general formula (I), acid addition salts with mineral acids such as hydrochloric acid, hydrobromic acid, hydroiodic acid, sulfuric acid, nitric acid, and phosphoric acid; organic acids such as formic acid, acetic acid, propionic acid, oxalic acid, malonic acid, succinic acid, fumaric acid, maleic acid, malic acid, tartaric acid, methanesulfonic acid, and ethanesulfonic acid; acidic amino acids such as aspartic acid and glutamic acid and the like can be mentioned. These salts also include solvate products thereof. In a compound represented by the general formula (I), an asymmetric carbon atom and a double bond can be present; in the present invention, such compounds can include an optical isomer and a geometric isomer. These isomers can be isolated and purified by a method known per se; in the present invention, the individual isomers can be used both in the form of a mixture and in an isolated state. Particularly preferred in the present invention is a compound represented by the general formula (I) wherein the ring A is a piperidine ring whose nitrogen atom is substituted by a lower alkyl group, particularly by an alkyl group having 1 to 4 carbon atoms, X is an oxygen atom, R¹ is a lower alkyl group, particularly an alkyl group having 1 to 4 carbon atoms, each of R², R³ and R⁴ is a hydrogen atom, and Y is a carbonyl group or a vinylidene group, particularly 2,8-dimethyl-3-methylene-1-oxa-8-azaspiro[4,5]decane or 2-ethyl-8-methyl-1-oxa-8-azaspiro[4,5]decan-3-one, more particularly (-)- (S)-2,8-dimethyl-3-methylene-1-oxa-8-azaspiro[4,5]decane L-tartrate monohydrate.

A compound represented by the general formula (I) can be produced as described in JP-B-HEI-5-44948 or/and WO92/20683. For example, the compound can be produced by carrying out a condensation cyclization reaction of a heterocyclic ylidene acetic acid ester having a heterocycle corresponding to the ring A protected appropriately, and a hydroxy (or mercapto) alkylcarboxylic acid (or thiocarboxylic acid) ester, and, if required, eliminating the protecting group. The compound can also be produced by treating an alkenyl-substituted heterocyclic alcohol with iodine, and, if required, eliminating the protecting group. Being the most preferable of the compounds represented by the general formula (I), (-)-(S)-2,8-dimethyl-3-methylene-1-oxa-8-azaspiro[4,5]decane L-tartrate monohydrate can be produced as described in Example 5 of JP-B-HEI-5-44948 and Example 1 of WO92/20683.

The percutaneously absorbable preparation of the present invention may be any preparation capable of delivering the present compound to the topical tissue of the eye when administered to a skin surface including the eyelid; for example, external preparations such as adhesive preparations, ointments, gels, and creams can be mentioned, with preference given to adhesive preparations, ointments, and gels. Because the ointment of the present invention is administered to the eyelid skin surface, it is clearly distinct from ocular ointments to be applied directly to the conjunctival sac with the intent of direct application to the eye, in terms of the method of administration and the site of administration. As used herein, an adhesive preparation means a preparation to be applied to the skin, such as a cataplasm, a patch, a tape, and a plaster.

In the present invention, the eyelid skin surface means the skin surface of the upper eyelid and the lower eyelid and the vicinity thereof.

The percutaneously absorbable preparation of the present invention is capable of sustainedly promoting lacrimation when the choice and quantity and the like of the present compound to be contained in the preparation are adjusted. The percutaneously absorbable preparation of the present invention permits an easy adjustment of a dosage of the present compound that is effective in promoting lacrimation.

To the percutaneously absorbable preparation of the present invention, as required, as long as the effect of the present invention is not affected, an optionally chosen ingredient in common use for pharmaceutical making can be added as an additive as appropriate. For example, ointment bases, gel bases, base matrixes for adhesive preparations, solvents, oils, surfactants, gums, resins, absorption enhancers, wetting agents and the like can be mentioned.

As examples of ointment bases, oil/fat bases such as petrolatum, paraffin, plastibase, silicone, vegetable oils, lard, waxes, and simple ointments; emulsion bases such as hydrophilic ointment (vanishing cream), hydrophilic petrolatum, purified lanolin, absorption ointments, hydrous lanolin, and hydrophilic plastibase (cold cream); and the like can be mentioned.

As examples of gel bases, thickening polymers such as carboxyvinyl polymer, polyacrylic acid, sodium polyacrylate, methylcellulose, polyvinyl alcohol, polyvinylpyrrolidone, polyethylene oxide, polyacrylamide, sodium alginate, gelatin, gum arabic, tragacanth gum, guar gum, xanthane gum, agar, carrageenan, and chitosan; fatty acid esters such as isopropyl myristate, isopropyl palmitate, and oleic propyleneglycol; fatty acids such as lactic acid, lauric acid, oleic acid, linoleic acid, and linolenic acid; aliphatic alcohols such as lauryl alcohol and oleyl alcohol; hydrocarbons such as squalene and squalane; and the like can be mentioned.

As examples of solvents, purified water, ethanol, lower alcohols, ethers, pyrrolidones, ethyl acetate and the like can be mentioned.

As oils, volatile and nonvolatile oils, solvents, resins and the like in common use in dermal external preparations can be mentioned, and these can be used in the form of any of liquids, pastes, and solids at room temperature. For example, higher alcohols such as cetyl alcohol and isostearyl alcohol; fatty acids such as isostearic acid and oleic acid; polyalcohols such as glycerin, sorbitol, ethylene glycol, propyleneglycol, and polyethylene glycol; esters such as myristyl myristate, hexyl laurate, decyl oleate, isopropyl myristate, and monostearic glycerin; and the like can be mentioned.

As examples of surfactants, anionic surfactants, cationic surfactants, nonionic surfactants, and amphoteric surfactants can be used.

As examples of anionic surfactants, fatty acid salts, alkylsulfates, polyoxyethylene alkylsulfates, alkylsulfocarboxylates, alkyl ether carboxylates and the like can be mentioned.

As examples of cationic surfactants, amine salts, quaternary ammonium salts and the like can be mentioned.

As examples of nonionic surfactants, polyoxyethylene hydrogenated castor oil, polyoxyethylene fatty acid esters, polyoxyethylene alkyl ethers, polyoxyethylene sorbitan fatty acid esters and the like can be mentioned.

As examples of amphoteric surfactants, alkylbetaine, dimethylalkylglycine, lecithin and the like can be mentioned.

As examples of gums and resins, sodium polyacrylate, cellulose ether, calcium alginate, carboxyvinyl polymer, ethylene-acrylic acid copolymer, vinylpyrrolidone-based polymer, vinyl alcohol-vinylpyrrolidone copolymer, nitrogen-substituted acrylamide-based polymer, polyacrylamide, cationic polymers such as cationized guar gum, dimethylacrylammonium-based polymer, acryl acrylate-methacrylate copolymer, polyoxyethylene-polypropylene copolymer, polyvinyl alcohol, pullulan, agar, gelatin, tamarind seed polysaccharide, xanthane gum, carrageenan, chitosan, high-methoxyl pectin, low-methoxyl pectin, guar gum, gum arabic, crystalline cellulose, arabinogalactane, karaya gum, tragacanth gum, alginic acid, albumin, casein, curdlan, gellan gum, dextran, cellulose, polyethyleneimine, highly polymerized polyethylene glycol, cationized silicone polymer, synthetic latex, acrylsilicone, trimethylsiloxysilicic acid, fluorinated silicone resin and the like can be mentioned.

As examples of absorption enhancers, 1-dodecylazacycloheptan-2-one, pyrothiodecane, oleyl alcohol, lauric acid, oleic acid, sodium lauryl sulfate, d-limonene, 1-menthol, 2-pyrrolidone, 1-methyl-2-pyrrolidone, N,N-dimethylformamide, N,N-dimethylacetamide, dimethylsulfoxide, decylmethylsulfoxide, N-lauroylsarcosine, isopropyl myristate, isopropyl palmitate, fumaric acid, maleic acid, sorbic acid, glycyrrhizic acid, myristyl lactate, cetyl lactate, polyoxyethylene oleyl ether, diethanolamide laurate, polyalcohols, glycerin, propyleneglycol, diethanolamine, triisopropanolamine, triethanolamine and the like can be mentioned. These may be used in combination of 2 kinds or more. Isopropyl myristate is preferable.

As examples of base matrixes for adhesive preparations, acrylic adhesives, silicone-based adhesives, rubber-based adhesives and the like can be mentioned; one can be chosen from among these substances as appropriate. The matrix may also be used in a state retained on one face of a support in common use for preparations to be applied to the skin, such as tape preparations, patches, cataplasms, and plasters, or another support of a material that does not interfere with the use of the present invention.

As examples of acrylic adhesives, acrylic acid-acrylic acid octyl ester copolymer, acrylic acid ester-vinyl acetate copolymer, 2-ethylhexyl acrylate-vinylpyrrolidone copolymer, butyl methacrylate-acrylate copolymer and the like can be mentioned.

As examples of silicone-based adhesives, polymethylphenylsiloxane copolymer, acrylic acid-dimethylsiloxane copolymer and the like can be mentioned.

As examples of rubber-based adhesives, styrene-isoprene-styrene copolymer, styrene-isoprene-styrene block copolymer, natural rubber, polyisobutylene, polybutene, ethylene-vinyl acetate copolymer (EVA) and the like, with a tackifier resin, a softening agent and the like added thereto as required, and the like can be mentioned.

As examples of wetting agents, glycerin, polyethylene glycol, sorbitol, maltitol, propyleneglycol, 1,3-butanediol, hydrogenated maltose syrup and the like can be mentioned.

The percutaneously absorbable preparation of the present invention can be produced by a conventional method. For example, in the case of an ointment, the ointment can be produced by mixing well the present compound and an ointment base, and, as required, a solvent, an oil, a surfactant, a gum, a resin, an absorption enhancer, a wetting agent and the like. In the case of a gel, the gel can be produced by adding a solvent to the gel base, neutralizing the resulting mixture with a pH adjuster, mixing the mixture with a solvent, an oil, a surfactant, a gum, a resin, an absorption enhancer, a wetting agent and the like added as required, adding thereto the present compound, and thoroughly kneading the mixture. In the case of an adhesive preparation (cataplasm, patch, tape, plaster), the adhesive preparation can be produced by adding to the present compound a base matrix and/or a gum, and, as required, a solvent, an oil, a surfactant, a resin, an absorption enhancer, a wetting agent and the like, mixing the ingredients thoroughly, spreading the resulting ointment material over a support such as a nonwoven cloth, a woven cloth, a plastic film (including a sheet) or a film comprising a combination thereof, coating the material with a release liner, or spreading the material over a release liner, and pressure-bonding the material onto the aforementioned support. The support preferably has a flexibility that allows application to the eyelid skin surface, and a thickness thereof is chosen as appropriate according to dosage form, preferably in the range of 10 to 3000 µm considering preparation toughness, sensation of discomfort upon application, and close contact quality. The percutaneously absorbable preparation of the present invention, which comprises a heterocyclic spiro compound or a salt thereof, may be supplemented not only with the above-described ingredients, but also with a stabilizer, an antioxidant, an antiseptic, a crosslinking agent, a pH adjuster, an ultraviolet absorbent and the like, as long as the effect of the present invention is not affected.

The present compound is used in a proportion of generally 0.1 to 60 parts by weight, preferably 1 to 40 parts by weight, more preferably 5 to 40 parts by weight, to 100 parts by weight of the base.

Preferably, the amount of the present compound contained in the percutaneously absorbable preparation of the present invention is generally 0.1 to 40% by weight, more preferably 1 to 30% by weight, particularly preferably 5 to 30% by weight. When the percutaneously absorbable preparation of the present invention is used as an adhesive preparation, the amount of the present compound contained is preferably in the range of 1 to 40% by weight, more preferably 5 to 30% by weight, particularly preferably 15 to 30% by weight. When the percutaneously absorbable preparation of the present invention is used as an ointment or a gel, the amount of the present compound contained is preferably in the range of 0.1 to 40% by weight, more preferably 1 to 30% by weight, particularly preferably 5 to 30% by weight.

The amount of absorption enhancer contained in the percutaneously absorbable preparation of the present invention is generally 1 to 60% by weight, preferably 5 to 50% by weight, particularly preferably 10 to 40% by weight. When the percutaneously absorbable preparation of the present invention is used as an adhesive preparation, the amount of absorption enhancer contained is preferably in the range of 5 to 60% by weight, more preferably 10 to 50% by weight, particularly preferably 20 to 40% by weight. When the percutaneously absorbable preparation of the present invention is used as an ointment or a gel, the amount of absorption enhancer contained is preferably in the range of 1 to 60% by weight, more preferably 5 to 50% by weight, particularly preferably 10 to 40% by weight.

The content ratio of the present compound and the absorption enhancer in the percutaneously absorbable preparation of the present invention is generally in the range of 1 to 20 parts by weight of the absorption enhancer to 1 part by weight of the present compound, preferably 1 to 10 parts by weight of the absorption enhancer to 1 part by weight of the present compound, more preferably 1 to 5 parts by weight of the absorption enhancer to 1 part by weight of the present compound. When the percutaneously absorbable preparation of the present invention is used as an adhesive preparation, the content ratio of the present compound and the absorption enhancer is preferably in the range of 1 to 20 parts by weight of the absorption enhancer to 1 part by weight of the present compound, more preferably 1.5 to 10 parts by weight of the absorption enhancer to 1 part by weight of the present compound, particularly preferably 2 to 10 parts by weight of the absorption enhancer to 1 part by weight of the present compound. When the percutaneously absorbable preparation of the present invention is used as an ointment or a gel, the content ratio of the present compound and the absorption enhancer is preferably in the range of 1 to 20 parts by weight of the absorption enhancer to 1 part by weight of the present compound, more preferably 1 to 10 parts by weight of the absorption enhancer to 1 part by weight of the present compound, particularly preferably 1 to 5 parts by weight of the absorption enhancer to 1 part by weight of the present compound.

As long as the accomplishment of any object of the present invention is not hampered, the percutaneously absorbable preparation of the present invention may be formulated with pharmaceutical ingredients other than the present compound, for example, steroidal or non-steroidal anti-inflammatory agents, antibacterial agents, anti-allergic agents, antihistaminic agents, antiviral agents, vasoconstrictors, cataract remedies, glaucoma remedies, mydriatic agents and the like.

In the percutaneously absorbable preparation and pharmaceutical composition of the present invention, the dosage of the present compound varies depending on the patient's condition and age, dosage form and the like, and is generally about 0.01 mg to 10 g/day, preferably about 0.1 mg to 1 g/day, more preferably about 1 mg to 0.2 g/day, as daily dosage for an adult, and this is administered in 1 to 5 divided portions as required.

Because the percutaneously absorbable preparation of the present invention is capable of sustainedly promoting lacrimation, and produces less and milder adverse reactions such as miosis than eyedrops, it is useful as a prophylactic/therapeutic agent for dry eyes, for example, dry eyes associated with ocular diseases such as lacrimal hyposecretion, dry eye syndrome, Sjogren's syndrome, keratoconjunctivitis sicca, Stevens-Johnson syndrome, blepharitis, and meibomitis, dry eyes associated with VDT (visual display terminal) work and wearing of contact lenses, and the like.

The subjects of administration being not limited, the percutaneously absorbable preparation and pharmaceutical composition of the present invention are useful in the treatment of dry eyes and others in various mammals such as humans, monkeys, mice, rats, guinea pigs, rabbits, pigs, dogs, horses, and bovines.

Furthermore, the present invention provides a commercial package comprising the percutaneously absorbable preparation of the present invention, and a written matter stating an explanation about the use of the preparation for the promotion of lacrimation and that the preparation produces less miosis than preparations for instillation.

In the commercial package of the present invention, as the written matter, what is called a package insert, which states points of note concerning intended use, indications, method of administration and the like, and the like can be mentioned.

### Examples

The present invention is hereinafter described in more detail by means of the following Examples and Test Examples, which, however, are for illustrative purposes only and never limit the scope of the invention.

In the Examples and Test Examples below, as the heterocyclic spiro compound, (-)-(S)-2,8-dimethyl-3-methylene-1-oxa-8-azaspiro[4,5]decane L-tartrate monohydrate (hereinafter referred to as Compound A) was used.

| (Example 1: An adhesive preparation containing Compound A) | |
|---|---|
| Compound A | 0.6 g |
| Isopropyl myristate | 2.4 g |
| Acrylic copolymer (PE-300) | 2.97 g (as solid content) |
| Polyisocyanate compound (CK401) | 0.003 g (as solid content) |
| Ethyl acetate | Appropriate amount |
| Total quantity | 6 g |

About 2 mL of ethyl acetate was added to Compound A previously milled in a mortar, these ingredients were mixed together and then sonicated in a disposable cup for about 30 seconds to uniformly disperse the Compound A, after which isopropyl myristate (produced by Wako Pure Chemical Industries, Ltd.) was added, and the ingredients were thoroughly mixed. Next, 7.425 g of a acrylic copolymer which is an adhesive base (PE-300, solid content of about 40% (in ethyl acetate/toluene), produced by Nippon Carbide Industries Co., Ltd.) and 0.03 g of a polyisocyanate compound which is a crosslinking agent (CK401, solid content of about 10% (in toluene), produced by Nippon Carbide Industries Co., Ltd.) were added in sequence, and the ingredients were thoroughly mixed. After degassing, the mixture was spread over a release liner using a doctor knife or a Baker applicator, and allowed to stand until the organic solvent volatilized. Subsequently, a support was placed on the liner, and bonded under pressure using a roller, and the support and liner was kept in a constant-temperature chamber at about 40°C for 8 to 12 hours to cause crosslinking, and the resulting product was used as an adhesive preparation containing Compound A.

| (Example 2: An ointment containing Compound A) | |
|---|---|
| Compound A | 0.4 g |
| Isopropyl myristate | 0.8 g |
| White petrolatum | 0.8 g |
| Total quantity | 2g |

White petrolatum and isopropyl myristate were mixed well according to the formulation shown above, Compound A was added to the resulting mixed ointment base, these ingredients were kneaded well, and the resulting product was used as an ointment containing Compound A.

| (Example 3: A gel containing Compound A) | |
|---|---|
| Compound A | 0.3 g |
| Isopropyl myristate | 1.2 g |
| 2% carboxyvinyl polymer gel | 1.5 g |
| Total quantity | 3 g |

1 g of carboxyvinyl polymer is added little by little to 49 mL of purified water, and thoroughly dispersed and swollen, after which 1 mL of 8 N NaOH is added to neutralize the mixture, and this mixture is used as a transparent 2% carboxyvinyl polymer gel base. Isopropyl myristate is added to 1.5 g of this gel base, and the ingredients are mixed. Furthermore, Compound A is added, the ingredients are thoroughly kneaded, and this mixture is used as a gel containing Compound A.

| (Example 4: A cataplasm containing Compound A) | |
|---|---|
| Compound A | 0.3 g |
| Sodium polyacrylate | 0.45 g |
| Glycerin | 0.3 g |
| Mentha oil | 0.01 g |
| Purified water | Appropriate amount |
| Total quantity | 3 g |

Purified water is mixed well in sodium polyacrylate and glycerin to obtain a hydrated ointment material. Furthermore, mentha oil and Compound A are added, and the ingredients are thoroughly kneaded. The resulting mixed ointment material is spread and shaped over a support (polyester nonwoven cloth and the like), a release liner is placed thereon, and the resulting product is used as a cataplasm containing Compound A.

| (Example 5: A tape containing Compound A) | |
|---|---|
| Compound A | 0.3 g |
| Isopropyl myristate | 1.2 g |
| Acrylic copolymer (PE-300) | 1.485 g (as solid content) |
| Polyisocyanate compound (CK401) | 0.0015 g (as solid content) |
| Ethyl acetate | Appropriate amount |

| | |
|---|---|
| Total quantity 3 g | |

About 2 mL of ethyl acetate is added to Compound A previously milled in a mortar, the ingredients are mixed together, and then sonicated in a disposable cup for about 30 seconds to uniformly disperse the Compound A, after which isopropyl myristate (produced by Wako Pure Chemical Industries, Ltd.) is added, and the ingredients are mixed well. Next, 3.7125 g of an acrylic copolymer which is an adhesive base (PE-300, solid content of about 40% (in ethyl acetate/toluene), produced by Nippon Carbide Industries Co., Ltd.), 0.015 g of a polyisocyanate compound which is a crosslinking agent (CK401, solid content of about 10% (in toluene), produced by Nippon Carbide Industries Co., Ltd.) are added in sequence, and the ingredients are mixed well. After degassing, the mixture is spread over a release liner using a doctor knife or a Baker applicator, and allowed to stand until the organic solvent volatilized. Subsequently, a support is placed on the liner, and bonded under pressure using a roller, and the support and liner is kept in a constant-temperature chamber at about 40°C for 8 to 12 hours to cause crosslinking, and the resulting product is used as a tape containing Compound A.

| (Example 6: An adhesive preparation containing Compound A) | |
|---|---|
| Compound A | 0.3 g |
| Isopropyl myristate | 0.6 g |
| Styrene-isoprene-styrene block copolymer | 1.05 g |
| Rosin ester-based tackifying resin | 1.05 g |
| Total quantity | 3 g |

Styrene-isoprene-styrene block copolymer, rosin ester-based tackifying resin and isopropyl myristate are dissolved in toluene, and Compound A is added to this solution. The resulting adhesive is coated over a silicone-treated PET (polyethylene terephthalate) film, and dried at 110°C for 3 minutes. Subsequently, another PET film is superposed, and the resulting product is used as an adhesive preparation containing Compound A.

| (Comparative Example 1: Base adhesive preparation) | |
|---|---|
| Isopropyl myristate | 1.2 g |
| Acrylic copolymer (PE-300) | 1.782 g (as solid content) |
| Polyisocyanate compound (CK401) | 0.0018 g (as solid content) |
| Ethyl acetate | Appropriate amount |
| Total quantity | 3 g |

Isopropyl myristate was weighed out in a disposable cup. Next, 4.455 g of an acrylic copolymer which is an adhesive base (PE-300, solid content of about 40% (in ethyl acetate/toluene), produced by Nippon Carbide Industries Co., Ltd.) and 0.018 g of a polyisocyanate compound which is a crosslinking agent (CK401, solid content of about 10% (in toluene), produced by Nippon Carbide Industries Co., Ltd.) were added in sequence, and the ingredients were mixed well. After degassing, the mixture was spread over a release liner using a doctor knife or a Baker applicator, and allowed to stand until the organic solvent volatilized. Subsequently, a support was placed on the liner, and bonded under pressure using a roller, and the support and liner was kept in a constant-temperature chamber at about 40°C for 8 to 12 hours to cause crosslinking, and the resulting product was used as a base adhesive preparation containing Compound A.

| (Comparative Example 2: An ophthalmic solution containing Compound A) | |
|---|---|
| Compound A | 1 g |
| Sodium dihydrogen phosphate dihydrate | 0.01 g |
| Sodium chloride | 0.09 g |
| Sodium hydroxide | Appropriate amount |
| Purified water | Appropriate amount |
| Total quantity | 10 mL (pH 7) |

Sodium dihydrogen phosphate dihydrate and sodium chloride were added to and dissolved in about 7 mL of purified water. Compound A was added to and dissolved in this liquid, and sodium hydroxide was added to obtain a pH of 7. Purified water was added to make a total quantity of 10 mL, and this solution was used as an ophthalmic solution containing Compound A.

| (Comparative Example 3: Base ointment) | |
|---|---|
| Isopropyl myristate | 0.8 g |
| White petrolatum | 1.2 g |
| Total quantity | 2 g |

White petrolatum and isopropyl myristate were mixed well according to the formulation shown above, and the resulting mixture was used as a base ointment.

| (Comparative Example 4: An ophthalmic solution containing Compound A) | |
|---|---|
| Compound A | 2 g |
| Sodium dihydrogen phosphate dihydrate | 0.01 g |
| Sodium chloride | 0.09 g |
| Sodium hydroxide | Appropriate amount |
| Purified water | Appropriate amount |
| Total quantity | 10 mL (pH 7) |

A solution was prepared according to the formulation shown above in the same manner as Comparative Example 2, and this solution was used as an ophthalmic solution containing Compound A.

### (Test Example 1: Tear secretion test (Schirmer's test))

### (Test methods)

Used in the test were rabbits (Japanese White, male, 9 to 10 weeks of age, 2.0 to 2.7 kg, purchased from Kitayama LABES Co., Ltd. and Fukusaki Rabbit Warren). Each rabbit had its hair removed previously in preparation for the application of a percutaneously absorbable preparation. Under general anesthesia with a combination of ketamine and xylazine, each rabbit had its hair removed around the eyelids and in the back using electrical clippers and a shaver in a way that did not hurt the skin. The adhesive preparations containing Compound A prepared in Example 1 were applied over an area of about 8 cm² (about 2 cm x about 4 cm) (in total about 16 cm²) in each of the upper eyelid and lower eyelid skins of one eye of each rabbit, whereas the opposite eye was kept untreated. Also applied to the back skin was the adhesive preparation containing Compound A prepared in Example 1 over an area of about 16 cm² (about 4 cm x about 4 cm). For a control group, the base adhesive preparation prepared in Comparative Example 1 was applied to the skins of the upper and lower eyelids as with the adhesive preparation containing Compound A of Example 1. Before application and after application, the amount of tear was measured over time using Schirmer paper (Whatman No. 41 filter paper used, produced by Showa Yakuhin Kako Co., Ltd.). Five minutes before measuring the amount of tear, 10 µL of 0.4% oxybuprocaine hydrochloride (Benoxil (registered trademark) eyedrop 0.4%, produced by Santen Pharmaceutical Co., Ltd.) was instilled to achieve local anesthesia, and the tear in the lower eyelid conjunctival sac was wiped off with filter paper, after which the amount of tear secreted in 1 minute was measured using Schirmer paper for the applied eyes in the group receiving the adhesive preparation containing Compound A of Example 1 administered in the upper and lower eyelid skins (n=3), the opposite eyes (no-application, n=3) and both eyes in the back administration group (n=6), and the applied eyes in the group receiving the base adhesive preparation of Comparative Example 1 administered in the upper and lower eyelid skins (n=3).

Also, 50 µL of the ophthalmic solution containing Compound A prepared in Comparative Example 2 was instilled to one eye, after which the same procedure was performed (10 minutes to 2 hours after instillation: n=8).

With the amount of tear secreted before application and before instillation as the reference (0 mm/min), increases in the amount of tear secreted after application and after instillation were measured.

### (Test results)

The results are shown in FIG. 1.

As shown in FIG. 1, in the applied eyes in the group receiving the preparation of Example 1 administered to the upper and lower eyelids, compared with the instilled eyes in the group receiving the preparation of Comparative Example 2, more sustained lacrimation was observed. In the applied eyes in the group receiving the preparation of Example 1 administered to the upper and lower eyelids, a larger amount of tear was secreted than in the back administration group. Furthermore, in the applied eyes in the group receiving the preparation of Example 1 administered to the upper and lower eyelids, a larger amount of tear was secreted than in the opposite eyes (no-application).

Judging from this fact, it is seen that the percutaneously absorbable ophthalmic preparation of the present invention is better than ophthalmic solutions for direct administration to the eyes in terms of sustained promotion of lacrimation. It is also seen that lacrimation is better promoted when the preparation of the present invention is administered to the eyelid skin surface than when administered to other sites. This suggests that the preparation might exhibit its lacrimation promoting effect when administered to the eyelid skin surface because Compound A transfers directly from the eyelid skin to the topical tissue of the eye, rather than transfers to the topical tissue of the eye via systemic circulation.

### (Test Example 2: Effects on pupil diameter)

### (Test methods)

Concurrently with Test Example 1, pupil diameters were measured. With the pupil diameters before application and before instillation as the reference (0 mm), changes in the pupil diameters after application and after instillation were measured. The measurements were performed using electronic digital calipers (MAX-CAL, produced by Nippon Sokutei Kogyo).

### (Test results)

The results are shown in FIG. 2.

As shown in FIG. 2, almost no miosis was observed in the applied eyes in the group receiving the preparation of Example 1 administered to the upper and lower eyelids. In the instilled eyes in the group receiving the preparation of Comparative Example 2, severe miosis was observed.

Judging from this fact, it is seen that the percutaneously absorbable ophthalmic preparation of the present invention produces less adverse reactions such as miosis and accompanying sensation of blackout than ophthalmic solutions for direct administration to the eyes.

### (Test Example 3: Tear secretion test (Schirmer's test))

### (Test methods)

Used in the test were rabbits (Japanese White, male, 9 to 10 weeks of age, 2.0 to 3.0 kg, purchased from Kitayama LABES Co., Ltd. and Fukusaki Rabbit Warren). Each rabbit had its hair removed previously in preparation for applying an ointment. Under general anesthesia with a combination of ketamine and xylazine, each rabbit had its hair removed around the eyelids using electrical clippers and a shaver in a way that did not hurt the skin. About 7 to about 8 mg (equivalent to an area of about 2 cm x about 4 cm = about 8 cm²) of the ointment containing Compound A prepared in Example 2 was applied to each of the skins of the upper eyelid and lower eyelid in the tape-stripped area of one eye of each rabbit, whereas the opposite eye was kept untreated. For a control group, the base ointment prepared in Comparative Example 3 was applied to the skins of the upper and lower eyelids in the tap-stripped area of each rabbit as with the ointment containing Compound A of Example 2. Before application and after application, the amount of tear was measured over time using Schirmer paper (Whatman No. 41 filter paper used, produced by Showa Yakuhin Kako Co., Ltd.). Five minutes before measuring the amount of tear, 10 µL of 0.4% oxybuprocaine hydrochloride (Benoxil (registered trademark) eyedrop 0.4%, produced by Santen Pharmaceutical Co., Ltd.) was instilled to achieve local anesthesia, and the tear in the lower eyelid conjunctival sac was wiped off with filter paper, after which the amount of tear secreted in 1 minute was measured using Schirmer paper for the applied eyes in the group receiving the ointment containing Compound A of Example 2 (1 hour after application: n=2; 2 to 6 hours after application: n=3) and the applied eyes in the group receiving the base ointment of Comparative Example 3 (n=2).

Also, 50 µL of the ophthalmic solution containing Compound A prepared in Comparative Example 4 was instilled to one eye, after which the same procedure was performed (n=3).

With the amount of tear secreted before application and before instillation as the reference (0 mm/min), increases in the amount of tear secreted after application and after instillation were measured.

### (Test results)

The results are shown in FIG. 3.

As shown in FIG. 3, a larger amount of tear was secreted in the applied eyes in the group receiving the preparation of Example 2 than in the instilled eyes in the group receiving the preparation of Comparative Example 4.

Judging from this fact, it is seen that the percutaneously absorbable ophthalmic preparation of the present invention is better than ophthalmic solutions for direct administration to the eyes in terms of lacrimation promotion.

### (Test Example 4: Effects on pupil diameter)

### (Test methods)

Concurrently with Test Example 3, pupil diameters were measured. With the pupil diameters before application and before instillation as the reference (0 mm), changes in the pupil diameters after application and after instillation were measured. The measurements were performed using electronic digital calipers (MAX-CAL, produced by Nippon Sokutei Kogyo).

### (Test results)

The results are shown in FIG. 4.

As shown in FIG. 4, almost no miosis was observed in the applied eyes in the group receiving the preparation of Example 2. In the instilled eyes in the group receiving the preparation of Comparative Example 4, severe miosis was observed.

Judging from this fact, it is seen that the percutaneously absorbable ophthalmic preparation of the present invention produces less adverse reactions such as miosis and accompanying sensation of blackout than ophthalmic solutions for direct administration to the eyes.

Judging from the above-described results of Test Examples 1 to 4, the percutaneously absorbable ophthalmic preparation of the present invention is a highly safe preparation that sustainedly promotes lacrimation by the Compound A contained therein, with less adverse reactions such as miosis, when administered to the eyelid skin surface.

### Industrial Applicability

According to the present invention, a percutaneously absorbable ophthalmic preparation comprising a heterocyclic spiro compound or a salt thereof, capable of promoting lacrimation when administered to the eyelid skin surface, and a method of promoting lacrimation by administering the percutaneously absorbable preparation to the eyelid skin surface can be provided. Furthermore, the percutaneously absorbable ophthalmic preparation of the present invention is a highly safe preparation that produces extremely fewer adverse reactions such as miosis observed with the use of preparations for direct administration to the eyes, such as ophthalmic solutions.

This application is based on Japanese Patent Application 2005-216442 filed in Japan on July 26, 2005, and all disclosures thereof are incorporated herein.

## Claims

1. A percutaneously absorbable ophthalmic preparation comprising as an active ingredient a heterocyclic spiro compound represented by the general formula (I): 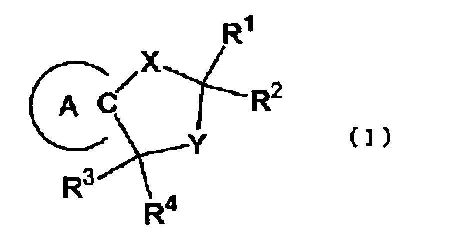 [wherein the ring A represents a piperidine ring whose nitrogen atom is optionally substituted by a lower alkyl group, a lower alkanoyl group, or a lower alkoxycarbonyl group, provided that the nitrogen atom of the piperidine ring and an optionally chosen non-spiro-bound carbon atom optionally form a bridge via a lower alkylene group,
X represents an oxygen atom or a sulfur atom,
Y represents a carbonyl group, a thiocarbonyl group, a group represented by the formula: 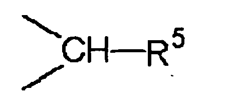 a group represented by the formula: 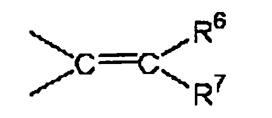 or a group represented by the formula: 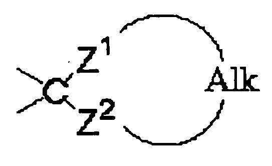 R¹, R² and R³ are the same or different, and each represents a hydrogen atom or a lower alkyl group,
R⁴ represents a hydrogen atom, a lower alkyl group, a carboxy group, a lower alkoxycarbonyl group, or a lower alkanoyl group, R⁵ represents a halogen atom, a hydroxyl group, a mercapto group, a lower alkoxy group, a lower alkylthio group, a lower alkanoyloxy group, or a lower alkanoylthio group,
R⁶ and R⁷ are the same or different, and each represents a hydrogen atom or a lower alkyl group,
Z¹ and Z² are the same or different, and each represents an oxygen atom or a sulfur atom,
Alk represents a lower alkylene group]
or a pharmaceutically acceptable salt thereof.

2. The percutaneously absorbable ophthalmic preparation according to claim 1, which is administered to the eyelid skin surface.

3. The percutaneously absorbable ophthalmic preparation according to claim 1 or 2, which promotes lacrimation.

4. The percutaneously absorbable ophthalmic preparation according to any one of claims 1 to 3, wherein the ring A in the general formula (I) is a piperidine ring whose nitrogen atom is substituted by a lower alkyl group, X is an oxygen atom, Y is a carbonyl group or a vinylidene group, R¹ is a lower alkyl group, and each of R², R³ and R⁴ is a hydrogen atom.

5. The percutaneously absorbable ophthalmic preparation according to any one of claims 1 to 3, wherein the active ingredient is (-)-(S)-2,8-dimethyl-3-methylene-1-oxa-8-azaspiro[4,5]decane L-tartrate monohydrate.

6. The percutaneously absorbable ophthalmic preparation according to any one of claims 1 to 5, wherein the amount of the active ingredient contained is 0.1 to 40% by weight.

7. The percutaneously absorbable ophthalmic preparation according to any one of claims 1 to 6, further comprising an absorption enhancer.

8. The percutaneously absorbable ophthalmic preparation according to claim 7, wherein the amount of the absorption enhancer contained is 1 to 60% by weight.

9. The percutaneously absorbable ophthalmic preparation according to claim 7 or 8, wherein the absorption enhancer is isopropyl myristate.

10. The percutaneously absorbable ophthalmic preparation according to any one of claims 1 to 9, which is an adhesive preparation.

11. The percutaneously absorbable ophthalmic preparation according to any one of claims 1 to 9, which is an ointment.

12. The percutaneously absorbable ophthalmic preparation according to any one of claims 1 to 9, which is a gel.

13. A commercial package comprising the percutaneously absorbable ophthalmic preparation according to any one of claims 1 to 12, and a written matter stating an explanation about the use of the preparation for promotion of lacrimation and that the preparation produces less miosis than preparations for instillation.

14. A method of promoting lacrimation by administering to the eyelid skin surface a percutaneously absorbable ophthalmic preparation comprising as an active ingredient a heterocyclic spiro compound represented by the general formula (I): 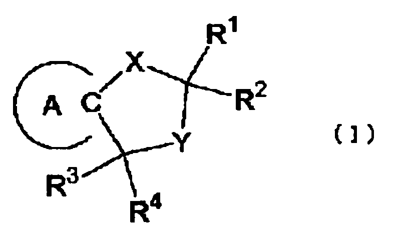 [wherein the ring A indicates a piperidine ring whose nitrogen atom is optionally substituted by a lower alkyl group, a lower alkanoyl group, or a lower alkoxycarbonyl group, provided that the nitrogen atom of the piperidine ring and an optionally chosen non-spiro-bound carbon atom optionally form a bridge via a lower alkylene group,
X represents an oxygen atom or a sulfur atom,
Y represents a carbonyl group, a thiocarbonyl group, a group represented by the formula: 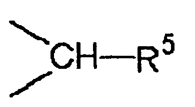 a group represented by the formula: 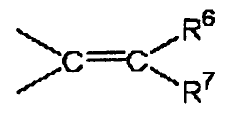 or a group represented by the formula: 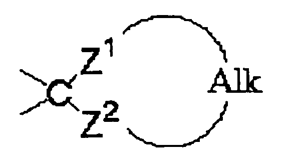 R¹, R² and R³ are the same or different, and each represents a hydrogen atom or a lower alkyl group,
R⁴ represents a hydrogen atom, a lower alkyl group, a carboxy group, a lower alkoxycarbonyl group, or a lower alkanoyl group, R⁵ represents a halogen atom, a hydroxyl group, a mercapto group, a lower alkoxy group, a lower alkylthio group, a lower alkanoyloxy group, or a lower alkanoylthio group,
R⁶ and R⁷ are the same or different, and each represents a hydrogen atom or a lower alkyl group,
z¹ and Z² are the same or different, and each represents an oxygen atom or a sulfur atom,
Alk represents a lower alkylene group]
or a pharmaceutically acceptable salt thereof.

15. Use of a heterocyclic spiro compound represented by the general formula (I): 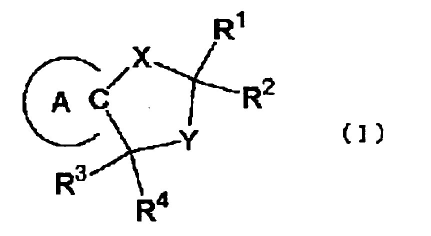 [wherein the ring A represents a piperidine ring whose nitrogen atom is optionally substituted by a lower alkyl group, a lower alkanoyl group, or a lower alkoxycarbonyl group, provided that the nitrogen atom of the piperidine ring and an optionally chosen non-spiro-bound carbon atom optionally form a bridge via a lower alkylene group,
X represents an oxygen atom or a sulfur atom,
Y represents a carbonyl group, a thiocarbonyl group, a group represented by the formula: 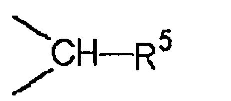 a group represented by the formula: 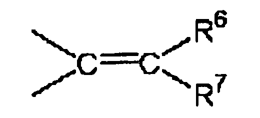 or a group represented by the formula: 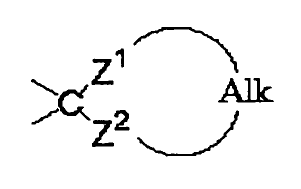 R¹, R² and R³ are the same or different, and each represents a hydrogen atom or a lower alkyl group,
R⁴ represents a hydrogen atom, a lower alkyl group, a carboxy group, a lower alkoxycarbonyl group, or a lower alkanoyl group, R⁵ represents a halogen atom, a hydroxyl group, a mercapto group, a lower alkoxy group, a lower alkylthio group, a lower alkanoyloxy group, or a lower alkanoylthio group,
R⁶ and R⁷ are the same or different, and each represents a hydrogen atom or a lower alkyl group,
Z¹ and Z² are the same or different, and each represents an oxygen atom or a sulfur atom,
Alk represents a lower alkylene group]
or a pharmaceutically acceptable salt thereof, for producing a percutaneously absorbable ophthalmic preparation.
